Europäisches Patentamt

European Patent Office

Office européen des brevets

(11) Veröffentlichungsnummer : **0 068 193**
**B1**

(12) **EUROPÄISCHE PATENTSCHRIFT**

(45) Veröffentlichungstag der Patentschrift :
02.05.84

(21) Anmeldenummer : 82105004.4

(22) Anmeldetag : 08.06.82

(51) Int. Cl.³ : **C 07 C 45/35, C 07 C 47/22,**
**B 01 J 37/02, B 01 J 27/18**

(54) Verfahren zur Herstellung von Acrolein bzw. Methacrolein durch katalytische Oxidation von Propylen bzw. Isobutylen oder tertiär-Butanol in sauerstoffhaltigen Gasgemischen.

(30) Priorität : 26.06.81 DE 3125061

(43) Veröffentlichungstag der Anmeldung :
05.01.83 Patentblatt 83/01

(45) Bekanntmachung des Hinweises auf die Patenterteilung : 02.05.84 Patentblatt 84/18

(84) Benannte Vertragsstaaten :
BE CH DE FR GB IT LI NL

(56) Entgegenhaltungen :
EP-A- 0 015 565
EP-A- 0 015 569
DE-A- 2 049 583
DE-A- 2 135 620
DE-A- 2 611 249
DE-A- 2 805 801
DE-B- 2 531 770
US-A- 4 267 386

(73) Patentinhaber : Degussa Aktiengesellschaft
Weissfrauenstrasse 9
D-6000 Frankfurt am Main 1 (DE)

(72) Erfinder : Arntz, Dietrich, Dr.
Amellastrasse 16
D-6450 Hanau (DE)
Erfinder : Prescher, Günter, Dr.
Liesingstrasse 2
D-6450 Hanau 9 (DE)
Erfinder : Hellos, Johannes
Westring 16
D-6453 Seligenstadt (DE)

Jouve, 18, rue St-Denis, 75001 Paris, France

Verfahren zur Herstellung von Acrolein bzw. Methacrolein durch katalytische Oxidation von Propylen bzw. Isobutylen oder tertiär-Butanol in sauerstoffhaltigen Gasgemischen

Die Erfindung betrifft ein Verfahren zur Herstellung von Acrolein bzw. Methacrolein durch Oxidation von Propylen bzw. Isobutylen oder tertiär-Butanol in sauerstoffhaltigen Gasgemischen an Schalenkatalysatoren, die an sich bekannte aktive Phasen enthalten, aber durch Herstellung nach einem neuen Verfahren verbesserte katalytische und mechanische Eigenschaften haben.

Es ist bekannt, daß durch Oxidation von Propylen bzw. Isobuten oder tertiär-Butanol mit molekularen Sauerstoff enthaltenden Gasen an oxidischen Katalysatoren Acrolein bzw. Methacrolein erhalten werden kann. Hierzu können Katalysatoren gemäß der DE-PS 2 049 583 verwendet werden, welche Oxide von Nickel, Kobalt, Eisen, Bismut, Phosphor, Molybdän sowie von seltenen Erden mit Zusätzen von anorganischen Trägersubstanzen enthalten.

Üblicherweise werden diese Katalysatoren in tablettierter oder strangverpresster Form eingesetzt. Beim technischen Einsatz solcher Katalysatoren ist es meist notwendig, sie durch Inertstoffe in der Schüttung zu verdünnen.

Diese Maßnahmen bereiten aber Probleme hinsichtlich der Homogenität der Schüttung und der Abführung der Reaktionswärmen aus inneren Bereichen der Schüttung an das Kühlmedium, was zu Selektivitäts- und damit Ausbeute-Einbußen führt.

Es ist schon seit 1963 bekannt, daß diesen Problemen mit sogenannten Schalenkatalysatoren, bei denen die aktive Katalysatorphase auf einen inerten Träger aufgezogen ist, begegnet werden kann. Durch die temperaturausgleichende Wirkung der Trägermasse werden lokale Überhitzungen vermieden. In den relativ dünnen Schalen aus aktivem Katalysatormaterial sind die Diffusionswege für die gasförmigen Reaktanten kurz und durch Variation der Schichtdicke des aktiven Materials läßt sich jede gewünschte Katalysatoraktivität einstellen (OE-PS 2 266 72).

Die DE-OS 2 611 249 behandelt den Einsatz eines solchen Schalenkatalysators für die Oxidation von Propylen zu Acrolein. Das dort beschriebene Verfahren gestattet indes nur die Herstellung von Schalenkatalysatoren mit relativ geringem Gehalt an aktiver Phase, so daß einem solchen Katalysator meist ein Vollkontakt, welcher durch und durch aus aktiver Masse besteht, nachgeschaltet werden muß, um wirtschaftlich befriedigende Umsätze zu erreichen. Die Schichtung von Katalysatoren unterschiedlicher Aktivität ist aber wegen der notwendigen Abstimmung der Einzelaktivitäten stets problematisch.

Die vorstehend beschriebenen bekannten Katalysatoren haben insbesondere den Nachteil, daß eine Verdünnung des Synthesegases mit beträchtlichen Mengen Wasserdampf erforderlich ist. Gerade der Wasserdampfgehalt im Synthesegas muß jedoch zur Erzielung wirtschaftlicher Reaktionsbedingungen verringert werden, was bei den bekannten technischen Katalysatoren nicht in befriedigendem Umfang möglich war (Shokubai, Bd. 19, 157-67 (1977) (Catalyst)).

In der EP-OS 0 015 569 wird nun ein Verfahren zur Herstellung von Acrolein bzw. Methacrolein durch Oxidation von Propylen bzw. Isobutylen in Sauerstoff enthaltenden Gasgemischen unter Verwendung eines Schalenkatalysators auch für geringe Wasserdampfgehalte des Synthesegases beschrieben. Die dafür vorgeschlagenen Katalysatoren werden so hergestellt, daß eine wässrige Suspension des katalytisch aktiven Materials auf bewegte Trägerteilchen aufgebracht wird, wobei man die Suspension in einer bestimmten gleichbleibenden Menge unter Teilabzug des Suspensionsmittels vermittels eines Gasstroms von 20-300 °C auf den Träger aufsprüht und wobei man eine im wesentlichen gleichbleibende Restfeuchte der Schale aufrechterhält.

Diesem und anderen, nach bekannten Verfahren erhältlichen Katalysatoren ist der Nachteil gemeinsam, daß bei dickeren Schalen, also Schalen, deren Gewichtsmenge, bezogen auf den Katalysator, 20 % übersteigt, die Abrieb- und Stoßfestigkeit der Schale für den Einsatz in großtechnischen Festbettreaktoren nicht vollständig befriedigt.

Insbesondere zeigte sich bei Schalenkatalysatoren, welche mit konventionellen Dragierkesseln oder Drehtellern gefertigt werden, welche nur ein Überleiten eines trocknenden Gasstroms über das bewegte Gut gestatten, eine Neigung zum Abplatzen der Schale bei Einwirkung von Temperaturgradienten.

Ferner kann mit solchen Vorrichtungen nur eine relativ breite Kornverteilung, die von der jeweiligen Schalendicke der Einzelpartikel des Katalysators bestimmt wird, erzielt werden.

Eine breite Kornverteilung bedeutet aber einerseits einen deutlich höheren Druckabfall von Katalysatorschüttungen und andererseits das Auftreten stark unterschiedlicher Wärmetönungen an den einzelnen Katalysatorpartikeln, was insgesamt zu einer Verschlechterung der Selektivität führt.

Das in der EP-OS 0 015 569 behandelte Katalysator-Herstellungsverfahren fordert die Aufrechterhaltung einer zeitlich konstantbleibenden Dosiergeschwindigkeit für Suspension und Trocknungsgas, um den Wassergehalt der entstehenden Schale während des Aufsprühens der Suspension praktisch konstant zu halten. Allerdings führt gerade diese Maßnahme dazu, daß mit zunehmender Dauer des Herstellungsganges die äußere Oberfläche der Schale zunehmend flüssigkeitsärmer wird, was das Auftragen dickerer Schichten mit ausreichender mechanischer Festigkeit erschwert oder verhindert.

Im übrigen wird durch die dort vorgesehene Führung des Trockengasstroms über die Oberfläche der Trägerschüttung hinweg nur eine mäßige Trocknungsgeschwindigkeit während der Ausbildung der Schale erreicht. Die Folge davon ist die bereits erwähnte nachteilige breite Kornverteilung.

Der Erfindung liegt die Aufgabe zugrunde, ein Verfahren zur Herstellung von Acrolein bzw. Methacrolein durch Oxidation von Propylen bzw. Isobutylen oder tertiär-Butanol in sauerstoffhaltigen Gasgemischen an abriebfesten Schalenkatalysatoren zu schaffen, wobei letztere aus einem oberflächlich rauhen, inerten Träger mit einer Korngröße von 0,5 bis 6 mm und einer diesen umhüllenden und in ihm verankerten Schale aus aktivem Katalysatormaterial bestehen, welche die Zusammensetzung $Ni_aCo_bFe_cBi_dP_eMo_fO_x$ hat, wobei a eine Zahl von 2-20, b eine Zahl von 0-15, a und b eine Zahl von 2-20, c eine Zahl von 0,1-7, d eine Zahl von 0,1-4, e eine Zahl von 0,1-4, f etwa 12 und x eine Zahl von 35-85 ist und in diesem Material zusätzlich 0,2 bis 5 Gew.% Tantal oder Samarium sowie gegebenenfalls noch 0,05-3,0 Gew.% Alkali- oder Erdalkalimetall, berechnet als Oxid, enthalten sind und dieses Material auf einer Trägersubstanz aus einem Schichtsilikat und/oder hochdispersem Siliciumdioxid — im ersten Fall in Gewichtsverhältnis 10 : 1 bis 1 : 1 — aufgebracht ist und wobei die Schale durch Aufsprühen einer Suspension des Ausgangsmaterials für die Schale auf eine bewegte Schüttung des Trägers unter Teilabzug des Suspensionsmittels durch einen Gasstrom von 20-250 °C unter Aufrechterhaltung einer im wesentlichen gleichbleibenden Restfeuchte der Schale sowie Trocknen und Tempern erhalten ist.

Diese Aufgabe wird erfindungsgemäß dadurch gelöst, daß Schalenkatalysatoren eingesetzt werden, bei deren Bereitung die Trägerschüttung durch mechanische Einwirkung in Mischbewegung versetzt und sie gleichzeitig durch Einblasen eines fluidisierenden, die Vermischung verstärkenden Gasstromes von unten her aufgelockert wird, dieser Schüttung im Gegenstrom zum Gas die ein Bindemittel und gegebenenfalls Porenbildner enthaltende Suspension einer Vorstufe des katalytisch aktiven Materials in einer mit anwachsender Dicke der Schale zunehmenden Menge zugeführt wird, wobei die Mengen von abgezogenem und aufgesprühtem Suspensionsmittel in einem weitgehend konstanten Verhältnis gehalten werden, das von der jeweils verwendeten Kombination aus Träger und Vorstufe bestimmt wird und wobei die Wärmeausdehnungskoeffizienten von Träger und Vorstufe so abgestimmt sind, daß sie sich im Höchstfall um 15 % unterscheiden, daß nach Beendigung des Aufsprühens die Schale durch Fortsetzen der verstärkten Mischbewegung verdichtet, dann die mechanische Mischbewegung eingestellt, das Gut im weiterströmenden Gas getrocknet und es schließlich, gegebenenfalls nach Zersetzen eines zugegebenen Porenbildners, getempert wird.

Dieses Beschichtungsverfahren für Trägerkörper sieht also vor, eine in Mischbewegung versetzte Trägerschüttung durch Einblasen eines Gasstroms von unten her aufzulockern, wobei der die fluidisierte Charge durchstreichende Gasstrom einen Teilabzug des Suspensionsmittels bewirkt. Zur Durchführung kommen entsprechend eingerichtete Mischaggregate in Betracht, wie z. B. spezielle Dragiertrommeln, Dragierkessel oder Drehteller. Bevorzugt sind Apparate, in denen eine gleichmäßige Durchströmung der Trocknungsluft durch die gesamte Schüttung erfolgt.

Eine bevorzugte Ausführungsform des erfindungsgemäßen Verfahrens sieht vor, daß die Schale des eingesetzten Schalenkatalysators in einer Vorrichtung gemäß DE-OS 2 805 801 aufgebracht und getrocknet ist.

Im sogenannten Driacoater, welcher im Gegenstromverfahren arbeitet, strömen Sprühflüssigkeit und Trockenluft in entgegengesetzter Richtung. Dieses u. a. in der genannten Offenlegungsschrift beschriebene Gerät besteht primär aus einer zylindrisch bzw. kegelig geformten und horizontal montierten Trommel. Über im Außenmantel der Trommel angebrachte Luftkanäle wird Trockenluft über auf der Trommelinnenwand angeordnete Hohlrippen, die auf der drehrichtungsabgekehrten Seite perforiert sind, ausschließlich von der Gutbettunterseite her eingebracht.

Beim Drehen der Trommel bewirken die wulstförmig gestalteten Hohlrippen und die durch diese eingeblasene Trocknungsluft die Fluidisierung und intensive Umwälzung des Schüttgutes ; dessen gleichmäßige Durchströmung durch die Trocknungsluft zeigt sich in einer gleichförmig ruhig abwärtsfließenden Eigenbewegung des Gutes. Die mit Feuchtigkeit beladene Abluft wird oberhalb der Schüttung über den hohlen Aufnahmedorn in der Drehachse der Trommel abgezogen.

Für das Versprühen der im erfindungsgemäßen Verfahren verwendeten Pulversuspensionen werden bevorzugt Zweistoffdüsen verwendet, mit denen einfacher als bei Einstoffdüsen die gewünschte Fördermenge unter beliebiger Feinverteilung bequem reguliert werden kann. Die Zerstäubung erfolgt üblicherweise mit Preßluft von 0,5-2 bar und in Abhängigkeit vom notwendigen Suspensionsdurchsatz (der sich aus der Chargengröße, der gewünschten Dicke des Pulverauftrages und der Präparationsdauer ergibt) mit ein oder mehreren Düsen von 2-4 mm Durchmesser bei Drucken der Suspension vor der Düse von 1-3 bar.

Bei Driacoater-Geräten mit Chargenkapazitäten von 10-200 l hat es sich als zweckmäßig erwiesen, den fluidisierenden Gasstrom auf einen spezifischen Durchsatz von 15-50 $Nm^3$/Stunde/l Träger einzustellen und ihn auf 60-100 °C aufzuheizen. Geringere Zuluftdurchsätze führen zu deutlich langsameren Trockengeschwindigkeiten, weniger gleichmäßiger Durchströmung der gesamten Schüttung durch Randgängigkeit an der Trommelwandung und damit wesentlich längeren Präparationszeiten. Zu hohe Zuluftdurchsätze verursachen dagegen ein zu starkes Austrocknen der Suspension auf dem Weg von der Düse zur Schüttungsoberfläche, wodurch ein Austrag von dabei ausgetrocknetem Vorstufen-Pulver mit der Abluft und eine nicht ausreichende Feuchte der Schale beim Auftrag verursacht wird. Es wurde gefunden, daß das Einhalten einer konstanten Feuchte der entstehenden Schale während des gesamten Schalenaufbaus eine wesentliche Voraussetzung zur Erzielung eines festhaftenden, fest im Trägermaterial verankerten Überzuges von aktivem Katalysatormaterial ist. Wird die Schale der Rohlinge während dieses Schalenaufbaus zu feucht, so erfolgt ein Agglomerieren mehrerer Körner miteinander. Bei zu trockenem Auftrag ist dagegen nicht die erwünschte Verankerung am Träger und auch keine gute Festigkeit der Schale zu erreichen. Eine wesentliche Erkenntnis besteht auch darin, daß sich bei Aufrecherhaltung einer bezüglich Temperatur und Menge konstanten Trocknungszuluft die erforderliche konstante Feuchte der Schale über die pro Zeiteinheit aufgesprühte Menge Suspension gut steuern laßt. Zur Sollwertvorgabe für eine solche Steuerung kann die Temperatur über der Schüttung oder die Feuchte der Abluft verwendet werden, die beide eine sensible Verfolgung des Trocknungsvorganges erlauben. Die günstigsten Sollwerte selbst sind abhängig von der Art des Pulvers und der Temperatur, der Feuchte und der Menge der Zuluft pro Volumeneinheit Trägermaterial. Je nach Feststoffgehalt der Suspension und Art der Vorstufe sollten 10-50 % des aufgesprühten Suspensionsmittels während deren Aufbau in der Schale bleiben. Es wurde gefunden, daß eine wesentliche Verbesserung der mechanischen Stabilität der Schale erreicht wird, wenn kein konstanter Sollwert, sondern eine fallende Temperatur bzw. eine steigende Abluftfeuchte vorgegeben wird. Dies ermöglicht durch eine entsprechende Programmsteuerung einen vollautomatischen Auftrag des Vorstufenpulvers.

Als Suspensionsmittel für die in Pulverform vorliegende Katalysatorstufe wird bevorzugt Wasser verwendet. Andere Flüssigkeiten wie z. B. Alkohole, sind nicht ausgeschlossen und haben in verschiedenen Punkten gegenüber Wasser Vorteile : Sie benötigen gegebenenfalls geringere Verdampfungsenergie oder erlauben eine bessere Anpassung von Benetzungs- und Löslichkeitsverhalten an die Vorstufe des katalytischen Materials und die Trägersubstanz. Letzteres ist bei wässrigen Suspensionen nur durch Zugabe von Bindemitteln beeinflußbar. Dem Vorteil organischer Lösungsmittel steht jedoch der Nachteil entgegen, mit der Trocknungsluft zündfähige Gemische zu bilden und spezielle Abluftreinigungsanlagen zu erfordern.

Der Festkörpergehalt der Suspension wird am besten so bemessen, daß die Suspension zu 20-80, vorzugsweise 40-70 Gew.% aus pulverförmiger Vorstufe besteht. Zu hohe Feststoffgehalte können Verstopfungen des Förder- und Sprühsystems für die Suspension verursachen. Zu geringe Feststoffgehalte erfordern dagegen unnötig verlängerte Präparationszeiten. Der empirisch ermittelbare, jeweils günstigste Feststoffgehalt hängt von den Eigenschaften der eingesetzten Vorstufe und ihrer Wechselwirkung mit dem Suspensionsmittel ab und liegt z. B. bei den im Rahmen der Beispiele für die Propenoxidation hergestellten Katalysatoren bei 55 %.

Es hat sich weiter gezeigt, daß eine deutliche Verbesserung der Abriebfestigkeit der Trägerkontakte durch die Verwendung von Bindemitteln, wie sie aus der Granulation bekannt sind, erreicht werden kann. Ihr Anteil an der Suspension ist abhängig von der Art des Bindemittels und liegt in der Regel zwischen 0,5 und 10 %. Während die untere Grenze fließend ist und bei der minimal notwendigen Menge zur gesicherten Verbesserung der Abriebfestigkeit liegt, wird bei zu hohen Bindemittelkonzentrationen die Trocknungsgeschwindigkeit während der Herstellung der Schale häufig verringert. Für die verwendeten Vorstufen der aktiven Katalysatorkomponente wurden die besten Ergebnisse mit 2-5 %, insbesondere ca. 4 Gew.% Glucose oder Harnstoff erreicht.

Bei der Oxidation von Propen zu Acrolein bzw. von Isobutylen oder tert.-Butanol zu Methacrolein wird insbesondere bei Verwendung von Schalenkatalysatoren mit hohem Anteil an aktiver Phase, d. h. dicken Schalen, häufig eine auf Porendiffusion zurückgehende Hemmung der Reaktion beobachtet. Es wurde nun gefunden, daß durch Zugabe von im Suspensionsmittel schwerlöslichen, feinteiligen Porenbildnern, wie Pentaerithrit, Polymethylmethacrylat, Polystyrol, Polyvinylalkohole o. ä., dieser hemmende Einfluß auf die Reaktion durch Ausbildung von Makroporen verringert werden kann. Der bevorzugte Gehalt der Suspension an Porenbildner beträgt 1-10 Gew.%. Voraussetzung für die Wirkung des Porenbildners ist, daß er unterhalb der Tempertemperatur durch Thermolyse oder Oxidation aus der aufgebauten Schale wieder entfernt werden kann.

Für den im erfindungsgemäßen Verfahren anzuwendenden Schalenkatalysator ist ausdrücklich vorgesehen, für den Schalenaufbau eine Vorstufe des katalytisch aktiven Materials heranzuziehen. Der Begriff « Vorstufe » ist so zu verstehen, daß das Vorstufenmaterial bereits alle Ingredienzien enthält, die für die Erzeugung des fertigen katalytisch aktiven Materials durch eine anschließende spezifische Wärmebehandlung benötigt werden.

Als Vorstufe des katalytisch aktiven Materials wird bevorzugt ein getrocknetes oder unterhalb der Tempertemperatur calciniertes Kopräzipitat aus vereinigten Salzlösungen der katalytisch aktiven Elemente eingesetzt.

Die Zusammensetzung dieses Kopräzipitats und seine spezielle Präparation ist nicht spezifisch für das erfindungsgemäße Verfahren, sondern abhängig von der gewünschten katalytischen Wirkung in der Reaktion, bei der der Schalenkatalysator eingesetzt wird. Üblicherweise kann die Präparation der Vorstufe analog der von bekannten Vollkatalysatoren erfolgen. Zur Erzielung einer guten Suspendierbarkeit der Vorstufe in dem Suspensionsmittel und einer störungsfreien Förderung der Suspension hat sich eine Kornverteilung von 1-150 μm mit einem vorzugsweisen Maximum im Bereich von 1,5-30 μm als zweckmäßig erwiesen.

Das zur Gewinnung des Schalenkatalysators angegebene Verfahren ermöglicht die Fertigung von Katalysatoren, bei denen die Menge der pulverförmigen Vorstufe das 0,1-2-fache des Trägergewichts beträgt, wobei sich dieser Bereich nicht durch spezifische Grenzen des Herstellungsverfahrens, sondern mehr aus praktischen Überlegungen zum Einsatz der erfindungsgemäßen Katalysatoren ergibt. Dies bedeutet, daß grundsätzlich auch Zusammensetzungen außerhalb des angegebenen Bereichs nach dem erfindungsgemäßen Verfahren herstellbar sind.

Es ist ferner ausdrücklich vorgesehen, die Wärmeausdehnungskoeffizienten von Träger und Vorstufe so abzustimmen, daß sie weitgehend übereinstimmen und sich im Höchstfall um nicht mehr als 15 % unterscheiden. Weichen nämlich diese Koeffizienten stärker voneinander ab, so reißt beim abschließenden Temperungsschritt die Schale.

Diese Risse können so groß werden, daß es zu einem schuppigen Abplatzen der Schale kommt. In jedem Fall ist mit dem Auftreten von Rissen eine starke Verringerung der mechanischen Stabilität der Schale, d. h. der Abriebfestigkeit verbunden.

Es wurde gefunden, daß eine Anpassung der Wärmeausdehnungskoeffizienten durch Auswahl eines geeigneten Trägers nur bedingt möglich ist und selten ausreicht, da die in Frage kommenden inerten Träger alle in dem relativ engen Bereich von $50\text{-}90 \cdot 10^{-7}/°C$, (für eindimensionale Ausdehnung) liegen.

Überraschend war nun die Feststellung, daß sich der Wärmeausdehnungskoeffizient des Vorstufenpulvers durch eine Temperaturvorbehandlung bei 250-600 °C auf den des Trägers abstimmen läßt. Die jeweils genauen Bedingungen hängen von der Zusammensetzung der Vorstufe und dem zu verwendenden Träger ab. Dabei ist darauf zu achten, daß diese Anpassung nicht für eine bestimmte Temperatur, sondern für den gesamten Temperaturbereich der abschließenden Temperung erfolgen muß (die bei dieser Temperung auftretenden Spannungen zwischen Schale und Träger sind für die eventuelle Rißbildung verantwortlich). Dadurch ist eine exakte Abstimmung, die eine fest definierte Bezugstemperatur voraussetzen würde, nicht möglich. Dies beruht insbesondere auf dem Umstand, daß bei den erfindungsgemäß anzuwendenden Materialien üblicherweise unterschiedliche Temperaturabhängigkeiten der Ausdehungskoeffizienten für Vorstufe und Träger vorliegen.

Nach dem erfindungsgemäßen Verfahren wird als Vorstufe für den Schalenkatalysator zur Herstellung von Acrolein oder Methacrolein aus Propylen, Isobutylen oder tertiär-Butanol ein oxidisches Pulver der Zusammensetzung $Ni_a$-

$Co_b Fe_c Bi_d P_e Mo_f O_x$, in dem a eine Zahl von 2-20, b eine Zahl von 0-15, a und b eine Zahl von 2-20, c eine Zahl von 0,1-7, d eine Zahl von 0,1-4, e eine Zahl von 0,1-4, f etwa 12 und x eine Zahl von 35-85 ist sowie zusätzlich 0,2-5 Gew.% Tantal oder Samarium,. berechnet als $Ta_2O_5$ oder $Sm_2O_3$ sowie gegebenenfalls noch 0,05-3,0 Gew.% Alkali- oder Erdalkalimetall, berechnet als Oxid, gegebenenfalls auf einer Trägersubstanz aus einem Schichtsilikat und/oder hochdispersem Siliciumdioxid — im ersten Fall im Gewichtsverhältnis 10 : 1 bis 1 : 1 — eingesetzt und der Schalenkatalysator 0,05-5 Stunden bei 520-650 °C getempert. Bei Einsatz von Alkali- oder Erdalkalimetall werden die Elemente K, Na und Mg bevorzugt.

Als vorteilhafte Trägermaterialien für die im Rahmen der Erfindung einzusetzenden Schalenkatalysatoren haben sich insbesondere α-Aluminiumoxid, Aluminiumsilikat, Magnesiumsilikat oder Siliciumcarbid erwiesen. An die Form der Träger werden keine speziellen Anforderungen durch das Verfahren gestellt. jedoch werden kugelförmige bevorzugt.

Porenfreies oder porenarmes Magnesiumsilikat oder Siliciumcarbid werden vor allem verwendet, wenn die aktive Phase nur oberflächlich auf den Träger aufgebracht und nicht in Hohlräumen des Trägers eingelagert werden soll. Das katalytische Material ist dagegen in den Hohlräumen von makroporösen α-Aluminiumoxiden und Alumosilikaten stärker geschützt, besser verankert und erfordert bei nicht zu starken Beschichtungen (kleiner 20 Gew.% aktive Phase) keine so harte Schale. Die Makroporen von Aluminiumsilikaten und α-Aluminiumoxid sollten im Bereich von 2-2 000, vorzugsweise 20-300 μm (90 %-Wert) liegen, um einerseits eine ausreichende Festigkeit des Trägers zu gewährleisten, andererseits aber das Ablagern der aktiven Phase in den Poren zu erlauben.

Vom Gesichtspunkt eines günstigen Verhaltens beim Schalenaufbau ergeben sich für die porenarmen bzw. -freien Träger Vorteile, da bei diesen zu Anfang der Präparation eine geringere Flüssigkeitsbeladung des Trägers auftritt und die bei makroporösen Trägern am Ende der Präparation aus den Poren austretende Feuchtigkeit beim Trocknungsprozeß schwieriger zu beherrschen ist.

Die Erfindung sieht auch vor, daß das Trägermaterial eine rauhe äußere Oberfläche haben soll, weil damit die Haftfestigkeit der Schale durch eine Tiefenverankerung des katalytisch aktiven Materials im Träger erhöht und ein gleichmäßiger Auftrag auf der gesamten Trägeroberfläche ermöglicht wird. Bei glatten Trägermaterialoberflächen wird meist ein schuppiger, unregelmäßig dicker Auftrag beobachtet. Als besonders günstig hat sich gezeigt, wenn die Trägeroberfläche eine Rauhigkeit, charakterisiert durch den Mittenrauhwert nach DIN 4768/1, gemessen mit dem Rauhtiefenmesser nach Hommel, von 5-50 μm aufweist.

Das Verfahren gemäß der Erfindung läßt sich unter Einsatz der vorstehend näher beschriebenen neuen Schalenkatalysatoren besonders günstig durchführen, wenn das der Oxidationsreaktion zugeführte sauerstoffhaltige Gasgemisch ein molares Verhältnis von Olefin bzw. Alkohol zu Sauerstoff und verdünnenden weitgehend inerten Bestandteilen von 1 : 1,5-2,5 : 7-20 hat.

Das sauerstoffhaltige Gasgemisch kann dabei als verdünnende, weitgehend inerte Bestandteile Stickstoff, Wasserdampf, Kohlenoxide und gesättigte aliphatische Kohlenwasserstoffe mit 1-5 C-Atomen oder deren Gemische enthalten.

Eine im Rahmen der Erfindung besonders bedeutende, weil ausbeuteverbessernde Betriebsweise sieht vor, die Oxidation von Propylen zu Acrolein bei einem molaren Verhältnis von Propylen : Sauerstoff : Stickstoff : Wasserdampf von 1 : 1,5-1,8 : 5,5-7 : 2-4 und bei einer spezifischen Belastung von 2-6 Mol Propylen/kg Schalenkatalysator · h zu betreiben.

Alternativ dazu kommt eine ebenfalls sehr vorteilhafte Fahrweise in Betracht, bei der die Oxidation von Propylen zu Acrolein unter teilweisem bis vollständigen Ersatz einer Wasserdampfeinspeisung durch ein inertes (aus der Reaktion stammendes oder extern zugeführtes) Gasgemisch bei einem molaren Verhältnis von Propylen : Luft : Inertgas : Wasser von 1 : 7-9 : 3-8 : 0-2 und bei einer spezifischen Belastung von 2-6 Mol Propylen/kg Schalenkatalysator · h durchgeführt wird.

Wenn man bei dieser Betriebsweise als Inertgas Abgas aus der Reaktion verwendet, so befreit man dieses vor der Rückführung in den Reaktor von Acrolein, Acrylsäure und anderen im Temperaturbereich von 0-40 °C kondensierbaren Bestandteilen. Günstig ist, wenn das Inertgas einen Wasserdampfgehalt von 0,5-7 Vol.% enthält.

Die Oxidation von Isobuten oder tertiär-Butanol zu Methacrolein wird demgegenüber am besten bei einem molaren Verhältnis von Isobuten bzw. tertiär-Butanol : Sauerstoff : Inertgas : Wasserdampf von 1 : 1,5-2,5 : 5,5-10 : 2-10 und bei einer spezifischen Belastung von 1-6 Mol Ausgangsverbindung/kg Schalenkatalysator · h durchgeführt.

Die Erfindung wird anschließend anhand von Ausführungsbeispielen weiter erläutert :

Beispiel 1

Das Kopräzipitat für die Herstellung der aktiven Katalysatorphase wird in der aus der DE-PS 2 049 583 bekannten Weise bereitet, indem einer Lösung von 32,3 kg Nickelnitrat $Ni(NO_3)_2 \cdot 6$ $H_2O$, 1 kg Kobaltnitrat $Co(NO_3)_2 \cdot 6 H_2O$ und 4,5 kg Eisennitrat $Fe(NO_3)_3 \cdot 9 H_2O$ in 38 kg Wasser nacheinander eine Lösung von 0,3 kg Samariumoxid $Sm_2O_3$ in 3,5 kg 38 %iger Salpetersäure, 5,8 kg Aerosil 200®, 10,8 kg Montmorillonit, eine Lösung von 23,4 kg Ammonmolybdat $(NH_4)_6Mo_7O_{24} \cdot 4 H_2O$ in 31,4 kg 3,5 %iger Phosphorsäure und eine Lösung von 5,4 kg Bismutnitrat $Bi(NO_3)_3 \cdot 5 H_2O$ in 4,5 kg 7,7 %iger Salpetersäure unter Rühren zugesetzt wird. Die erhaltene Suspension des Kopräzipitäts wird auf einem Walzentrockner getrocknet, bei 530 °C im Drehrohr calciniert und dann gemahlen. Das so erhaltenen Pulver der Vorstufe des katalytisch aktiven Materials hat eine Kornverteilung von 2-40 µm (> 90 %, Maximum bei 15 µm) und bei 400 °C einen Wärmeausdehnungskoeffizienten von $81 \cdot 10^{-7}/°C$.

Durch Aufschlämmen von 6,5 kg dieses Vorstufenpulvers in 4,7 kg Wasser unter Zusatz von 0,5 kg D-Glucose als Bindemittel und 0,3 kg Pentaerithrit (Typ R, Fa. Degussa) als Porenbildner wird die Suspension für das Ausgangsmaterial der Schale hergestellt. Als Träger zu diesem Vorstufenmaterial werden gebrannte Steatit-Kugeln mit 4 mm Durchmesser ausgewählt, die praktisch porenfrei sind, eine rauhe Oberfläche (Mittenrauhwert 25 µm nach DIN 4768/1) haben und deren longitudinaler Wärmeausdehnungskoeffizient bei 400 °C bei $90 \cdot 10^{-7}/°C$ liegt.

6 kg des Trägers werden in einen Driacoater 500 gegeben und in diesem durch Einblasen von 2 m³/Min. auf 80 °C vorgeheizter Luft und Drehen der Trommel mit 20 Umdrehungen pro Minute in intensive Misch- und Fließbewegung versetzt.

Auf den so bewegten Träger werden zunächst in 2 Minuten 0,4 l der Suspension mit einer Zweistoffdüse aufgesprüht. Das Aufsprühen der restlichen Suspension wird so über die Ablufttemperatur aus dem Kessel gesteuert, daß ständig eine gleichbleibende Feuchte der Schale beobachtet wird. Dabei fällt die Ablufttemperatur von anfänglich 48 °C auf 39 °C am Ende des Suspensionsauftrags (nach 60 Min.) ab und der Suspensionsauftrag steigt von 0,096 auf 0,104 l/Minute.

Nach Ende des Aufsprühvorganges schließt sich bei weiterdrehender Trommel eine Verdichtungsphase von 5 Minuten und dann eine 20-minütige Trockenphase bei nur einmaliger Kesselumdrehung pro Minute an. Nach Lufttrocknen über Nacht wird der Porenbildner im Drehrohr bei 400 °C und einer mittleren Verweilzeit von 15 Minuten zersetzt. Die Aktivierung des Katalysators erfolgt bei 550 °C und 15 Minuten Verweilzeit ebenfalls im Drehrohr.

Der erhaltene Schalenkatalysator hat eine harte, rissfreie Schale. Der mittlere Durchmesser der erhaltenen Schalenkatalysatoren beträgt 5,25 mm mit einer Standardabweichung von 0,3 mm. Der Abrieb wird im La-Roche-Friabilator durch Roll- und Fallverschleiß bei 20 Umdrehungen pro Minute als Abrieb von kleiner 2 mm nach 7 Minuten bestimmt. Er betrug für den getemperten Schalenkatalysator weniger als 0,2 Gew.%. Nach einer Temperaturbehandlung von 100 Zyklen Aufheizen und Abkühlen, in denen der Katalysator innerhalb von 0,5 Stunden jeweils von 250 °C auf 400 °C aufgeheizt und wieder auf 250 °C abgekühlt wurde, war der Wert nicht signifikant erhöht und lag bei 0,2 Gew.%.

Beim Falltest, freier Fall von 100 ml Kontakt durch ein 3,4 m langes Rohr mit 20 mm Innendurchmesser auf eine harte Unterlage, entstand ein Anteil an Bruch von < 2 mm von 0,03 Gew.%.

Beispiel 2

Die katalytische Wirkung des in Beispiel 1 hergestellten Katalysators wurde in einem technischen Reaktor-Rohr von 20,5 mm Innendurchmesser, das von außen durch in Salzbad gekühlt wird, bei einer Katalysatorschüttungslänge von 2,7 m anhand der Umsetzung von Propen zu Acrolein getestet.

a) Bei einer Einspeisung von 5 Mol Propen/Stunde, 40 Mol Luft/Stunde und 10,1 Mol $H_2O$/Stunde werden bei einer Salzbadtemperatur von 351 °C ein Umsatz von 94 %, eine Acrolein-Einsatzausbeute von 79,2 und eine Summenselektivität für Acrolein und Acrylsäure von 92,5 % erreicht.

b) Bei einer Einspeisung von 5 Mol Propen/Stunde, 38 Mol Luft/Stunde und 29 Mol rückgeführtem Abgas/Stunde (Zusammensetzung: 7 % $O_2$, 1 % Propen, 92 % Inertgas (Propen, Stickstoff, Kohlenstoffdioxid und Wasser)) werden bei einer Salzbadtemperatur von 355 °C ein Umsatz von 94,9 %, eine Acrolein-Ausbeute von 79,5 % und eine Selektivität für Acrolein und Acrylsäure von 92 % erhalten.

Beispiel 3

2 kg des in Beispiel 1 hergestellten Vorstufenpulvers werden in 1,9 kg Wasser unter Zusatz von 0,05 kg Glucose als Bindemittel suspendiert. Im Driacoater 500 werden 6 kg eines Aluminiumsilikatträgers mit einer spezifischen Oberfläche von kleiner 1 m²/g, einem Durchmesser von 4,8 mm, einer Makroporosität, bei der 90 % der Poren zwischen 70 und 500 µm liegen, einer oberflächlichen Rauhigkeit nach DIN 4768/1 mit einem Mittenrauhwert von 48 µm und einem Wärmeausdehnungskoeffizienten bei 400 °C von $70 \cdot 10^{-7}$/°C durch Einblasen von 2 m³/min auf 70 °C vorgeheizter Luft und durch Drehen der Trommel bei 12 Umdrehungen pro Minute in intensive Misch- und Fließbewegung versetzt und auf den so bewegten Träger in 35 Minuten die Suspension analog zu Beispiel 1 so aufgesprüht, daß die Ablufttemperatur von anfänglich 43 °C auf 38 °C absank. Nach Trocknen des Rohkatalysators wurde er bei 575 °C im Drehrohr aktiviert. Der Abrieb, gemessen im La-Roche-Friabilator betrug 0,2 Gew.%.

Beispiel 4

Ein Vorstufenpulver wurde entsprechend Beispiel 1 hergestellt, nur mit dem Unterschied, daß der Samariumoxidlösung zusätzlich 0,4 kg Kaliumnitrat zugesetzt wurden. Das bei 470 °C im Drehrohr calcinierte Vorstufenpulver hat einen Wärmeausdehnungskoeffizienten von $80 \cdot 10^{-7}$/°C. 9 kg dieses Vorstufenmaterials werden mit 0,7 kg Pentaerithrit (Porenbildner) und 0,8 kg Glucose (Bindemittel) in 5,3 kg Wasser suspendiert und im Driacoater auf 6 kg intensiv bewegten Steatit-Trägern (wie in Beispiel 1) aufgesprüht. Dabei wurden die 2,5 m³/min Zuluft auf 85 °C vorgewärmt und die innerhalb von 95 Minuten aufgesprühte Suspension so dosiert, daß die Ablufttemperatur von anfänglich 51 °C auf 42 °C absank. Nach Trocknen, Zersetzen von Porenbildner und Bindemittel bei 400 °C und Aktivieren bei 550 °C im Drehrohr hatte der Katalysator einen Abrieb im La-Roche-Friabilator von 0,3 Gew.%.

Beispiel 5

Ein Vorstufenpulver wurde analog zu Beispiel 1 hergestellt, indem einer Lösung von 6,7 kg Nickelnitrat $Ni(NO_3)_2 \cdot 6\,H_2O$, 12,3 kg Kobaltnitrat $Co(NO_3)_2 \cdot 6\,H_2O$ und 6,9 kg Eisennitrat $Fe_2(No_3)_3 \cdot 9\,H_2O$ in 30,4 kg Wasser nacheinander eine Lösung von 18,4 kg Ammoniummolybdat $(NH_4)_6Mo_7O_{24} \cdot 4\,H_2O$ in 24,1 kg 3,1 %iger Phosphorsäure, eine Lösung von 7 kg Bismutnitrat $Bi(No_3)_3 \cdot 5\,H_2O$ in 7,0 kg 0,8 %iger Salpetersäure und 6 kg pyrogene Kieselsäure (Aerosil® 200) unter Rühren zugesetzt wurde. Das entstehende Kopräzipitat wurde auf einem Walzentrockner bei 140 °C getrocknet und bei 535 °C im Drehrohr calciniert und dann in einer Stiftmühle gemahlen. Das erhaltene Pulver hatte eine Kornverteilung von 5-80 µm (90 %-Wert) mit einem Maximum bei 30 µm und einem Wärmeausdehnungskoeffizienten von $85 \cdot 10^{-7}$/°C.

Aus 7,5 kg dieses Vorstufenpulvers mit einem Zusatz von 0,6 kg Glucose und 0,5 kg Pentaerithrit in 6,2 kg Wasser und 6 kg Steatit-Träger wurde entsprechend Beispiel 1 ein abriebfester Schalenkatalysator im Driacoater 500 hergestellt. Der Abrieb im La-Roche-Friabilator betrug 0,25 Gew.%.

Beispiel 6

Ein Vorstufenpulver wurde wie in Beispiel 5 hergestellt, nur mit einer zusätzlichen Beigabe von 0,2 kg $KNO_3$ zur ersten Lösung. Das erhaltene Pulver hatte eine Kornverteilung von 3-70 µm (90 %-Wert) mit einem Maximum bei 25 µm und einem Wärmeausdehnungskoeffizienten von $84 \cdot 10^{-7}$/°C.

Aus 5,5 kg dieses Vorstufenpulvers mit 0,4 kg Glucose in 4,5 kg Wasser und 6 kg Steatit-Träger wurde entsprechend Beispiel 1 ein abriebfester Schalenkatalysator im Driacoater 500 hergestellt. Der Abrieb im La-Roche-Friabilator betrug 0,3 Gew.%.

Beispiel 7

50 ml des in Beispiel 5 hergestellten Katalysators wurden in einen von außen durch ein Salzbad auf 362 °C temperierten Rohrreaktor von 16 mm Innendurchmesser gefüllt. Bei einer Einspeisung pro Stunde von 0,25 Mol Propen, 45 Nl Luft und 9,5 g Wasser erhielt man einen Umsatz von 92,5 % eine Acroleinausbeute, bezogen auf eingesetztes Propen, von 80,5 % und eine Summenselektivität, bezogen auf eingesetztes Propen, von 95,8 %.

Beispiel 8

50 ml des im Beispiel 3 hergestellten Katalysators wurden in einen von außen durch ein Salzbad auf 370 °C temperierten Reaktor von 16 mm Innendurchmesser gefüllt. Bei einer Einspeisung pro Stunde von 0,15 Mol Isobuten, 35 Nl Luft und 10,5 g Wasser erhielt man einen Umsatz von 91 % und, bezogen auf eingespeistes Isobuten, eine Methacroleinausbeute von 74,1 % sowie eine Summenausbeute für Methacrolein und Methacrylsäure von 82,4 %.

Beispiel 9

50 ml des im Beispiel 4 hergestellten Katalysators wurden in einen von außen durch ein Salzbad auf 355 °C temperierten Reaktor von 16 mm Innendurchmesser gefüllt. Bei einer Einspeisung pro Stunde von 0,15 Molt t-Butanol, 35 Nl Luft und 10,5 kg Wasser erhielt man einen Umsatz von 92,8 % und, bezogen auf eingespeistes t-Butanol eine Methacroleinausbeute von 75,2 % und eine Summenausbeute für Methacrolein und Methacrylsäure von 81,9 %.

Beispiel 10

50 ml des im Beispiel 6 hergestellten Katalysators wurden wie im Beispiel 8 bei einer Salzbadtemperatur von 382 °C getestet. Der Umsatz lag bei 93,6 %, die auf eingespeistes Isobuten bezogene Methacroleinausbeute bei 75,6 % und die Summenselektivität für Methacrolein und Methacrylsäure bei 82,9 %.

**Ansprüche**

1. Verfahren zur Herstellung von Acrolein bzw. Methacrolein durch Oxidation von Propylen bzw. Isobutylen oder tertiär-Butanol in sauerstoffhaltigen Gasgemischen an abriebfesten Schalenkatalysatoren aus einem oberflächlich rauhen, inerten Träger mit einer Korngröße von 0,5 bis 6 mm und einer diesen umhüllendem und in ihm verankerten Schale aus aktivem Katalysatormaterial der Zusammensetzung $Ni_aCo_bFe_cBi_dP_eMo_fO_x$, in dem a eine Zahl von 2-20, b eine Zahl von 0-15, a und b eine Zahl von 2-20, c eine Zahl von 0,1-7, d eine Zahl von 0,1-4, e eine Zahl von 0,1-4, f etwa 12 und x eine Zahl von 35-85 ist, sowie zusätzlich 0,2 bis 5 Gew.% Tantal oder Samarium, berechnet als $Ta_2O_5$ oder $Sm_2O_3$, sowie gegebenenfalls noch 0,05-3,0 Gew.% Alkali- oder Erdalkalimetall, berechnet als Oxid, auf einer Trägersubstanz aus einem Schichtsilikat und/oder hochdispersem Siliciumdioxid — im ersten Fall im Gewichtsverhältnis 10 : 1 bis 1 : 1 — wobei die Schale durch Aufsprühen einer Suspension des Ausgangsmaterials für die Schale auf eine bewegte Schüttung des Trägers unter Teilabzug des Suspensionsmittels durch einen Gasstrom von 20-250 °C unter Aufrechterhaltung einer im wesentlichen gleichbleibenden Restfeuchte der Schale sowie Trocknen und Tempern erhalten ist, dadurch gekennzeichnet, daß Schalenkatalysatoren eingesetzt werden, bei deren Bereitung die Trägerschüttung durch mechanische Einwirkung in Mischbewegung versetzt und sie gleichzeitig durch Einblasen eines fluidisierenden, die Vermischung verstärkenden Gasstromes von unten her aufgelockert wird, dieser Schüttung im Gegenstrom zum Gas die ein Bindemittel und gegebenenfalls Porenbildner enthaltende Suspension einer Vorstufe des katalytisch aktiven Materials in einer mit anwachsender Dicke der Schale zunehmenden Menge zugeführt wird, wobei die Mengen von abgezogenem und aufgesprühtem Suspensionsmittel in einem weitgehend konstanten Verhältnis gehalten werden, das von der jeweils verwendeten Kombination aus Träger und Vorstufe bestimmt wird und wobei die Wärmeausdehnungskoeffizienten von Träger und getrockneter pulverförmiger Vorstufe so abgestimmt sind, daß sie sich im Höchstfall um 15 % unterscheiden, daß nach Beendigung des Aufsprühens die Schale durch Fortsetzen der verstärkten Mischbewegung verdichtet, dann die mechanische Mischbewegung eingestellt, das Gut im weiterströmenden Gas getrocknet und es schließlich, gegebenenfalls nach Zersetzen eines zugegebenen Porenbildners, vorzugsweise 0,05-5 Stunden bei 520-650 °C, getempert wird.

2. Verfahren nach Anspruch 1, dadurch gekennzeichnet, daß die Schale des eingesetzten Schalenkatalysators in einer Vorrichtung gemäß DE-OS 2 805 801 aufgebracht und getrocknet ist.

3. Verfahren nach Anspruch 1 oder 2, dadurch gekennzeichnet, daß der Schalenkatalysator unter Einstellung des fluidisierenden Gasstroms auf einen spezifischen Durchsatz von 15-50 $Nm^3$ pro Stunde und Liter Träger und unter Verwendung von Wasser als Suspensionsmittel erhalten ist.

4. Verfahren nach den Ansprüchen 1-3, dadurch gekennzeichnet, daß der Schalenkatalysator unter Einsatz einer Suspension erhalten ist, welche zu 20 bis 80, vorzugsweise 40-70, insbesondere 55 Gew.% aus der pulverförmigen Vorstufe besteht.

5. Verfahren nach den Ansprüchen 1-4, dadurch gekennzeichnet, daß der Schalenkatalysator unter Einsatz einer Suspension erhalten ist, die als Bindemittel 2-5, insbesondere um 4 Gew.% Glucose oder Harnstoff enthält.

6. Verfahren nach den Ansprüchen 1-5, dadurch gekennzeichnet, daß der Schalenkatalysator unter Einsatz einer Suspension des pulverförmigen Ausgangsmaterials für die Schale erhalten ist, die 1-10 Gew.%, bezogen auf das Gewicht dieses Materials, eines im Suspensionsmittel schwerlöslichen feinteiligen Porenbildners enthielt, welcher unterhalb der Tempertemperatur durch Thermolyse oder Oxidation entfernt wurde.

7. Verfahren nach den Ansprüchen 1-6, dadurch gekennzeichnet, daß der Schalenkatalysator unter Einsatz einer Vorstufe aus einem getrockneten oder unterhalb der Tempertemperatur calcinierten Kopräzipitat aus vereinigten Salzlösungen der katalytisch aktiven Elemente erhalten ist.

8. Verfahren nach den Ansprüchen 1-7, dadurch gekennzeichnet, daß die Vorstufe des eingesetzten Schalenkatalysators ein Pulver mit einer Kornverteilung von 1-150 μm mit einem vorzugsweisen Maximum im Bereich von 1,5-30 μm ist.

9. Verfahren nach den Ansprüchen 1-8, dadurch gekennzeichnet, daß die Menge der pulverförmigen Vorstufe des eingesetzten Schalenkatalysators das 0,1-2-fache des Trägergewichts beträgt.

10. Verfahren nach den Ansprüchen 1-9, dadurch gekennzeichnet, daß der Träger des eingesetzten Schalenkatalysators α-Aluminiumoxid, Aluminiumsilikat, Magnesiumsilikat oder Siliciumcarbid ist.

11. Verfahren nach Anspruch 10, dadurch gekennzeichnet, daß die Rauhigkeit der Trägeroberfläche einen Mittenrauhwert von 5 bis 50 μm nach DIN 4768/1, gemessen mit dem Rauhtiefenmesser nach Hommel, hat.

12. Verfahren nach den Ansprüchen 1-11, dadurch gekennzeichnet, daß zur Bereitung des Schalenkatalysators der Wärmeausdehnungskoeffizient des Vorstufenpulvers durch eine Temperaturvorbehandlung bei 250-600 °C auf den des Trägers abgestimmt ist.

13. Verfahren nach den Ansprüchen 1-12, dadurch gekennzeichnet, daß das der Oxidationreaktion an dem Schalenkatalysator zugeführte sauerstoffhaltige Gasgemisch ein molares Verhältnis von Olefin bzw. Alkohol zu Sauerstoff und verdünnenden weitgehend inerten Bestandteilen von 1 : 1,5-2,5 : 7-20 hat.

14. Verfahren nach Anspruch 13, dadurch gekennzeichnet, daß das sauertoffhaltige Gasgemisch als verdünnende, weitgehend inerte Bestandteile Stickstoff, Wasserdampf, Kohlenoxide und gesättigte aliphatische Kohlenwasserstoffe mit 1-5 C-Atomen oder deren Gemische enthält.

15. Verfahren nach den Ansprüchen 1-14, dadurch gekennzeichnet, daß die Oxidation von Propylen zu Acrolein bei einem molaren Verhältnis von Propylen : Sauerstoff : Stickstoff : Wasserdampf von 1 : 1,5-1,8 : 5,5-7 : 2-4 und bei einer spezifischen Belastung von 2-6 Mol Propylen/kg Schalenkatalysator · h betrieben wird.

16. Verfahren nach den Ansprüchen 1-14, dadurch gekennzeichnet, daß die Oxidation von Propylen zu Acrolein unter teilweisen bis vollständigen Ersatz einer Wasserdampfeinspeisung durch ein inertes Gasgemisch bei einem molaren Verhältnis von Propylen : Luft : Inertgas : Wasser von 1 : 7-9 : 3-8 : 0-2 und bei einer spezifischen Belastung von 2-6 Mol Propylen/kg Schalenkatalysator · h durchgeführt wird.

17. Verfahren nach Anspruch 16, dadurch gekennzeichnet, daß als Inertgas das von Acrolein, Acrylsäure und anderen im Temperaturbereich von 0-40 °C kondensierbaren Bestandteilen befreite Abgas aus der Reaktion verwendet wird.

18. Verfahren nach Anspruch 16 oder 17, dadurch gekennzeichnet, daß das Inertgas 0,5-7 Vol.% Wasserdampf enthält.

19. Verfahren nach den Ansprüchen 1-14, dadurch gekennzeichnet, daß die Oxidation von Isobuten oder tertiär-Butanol zu Methacrolein bei einem molaren Verhältnis von Isobuten bzw. tertiär-Butanol : Sauerstoff : Inertgas : Wasserdampf von 1 : 1,5-2,5 : 5,5-10 : 2-10 und bei einer spezifischen Belastung von 1-6 Mol Ausgangsverbindung/kg Schalenkatalysator · h durchgeführt wird.

## Claims

1. A process for the production of acroleine or methacroleine by the oxidation of propylene or isobutylene or tertiary butanol in gas mixtures containing oxygen on abrasion-resistant shell catalysts consisting of a rough-surface, inert carrier having a grain size of from 0.5 to 6 mm, and a shell which encloses the carrier and is anchored thereto, consisting of active catalyst material having the composition $Ni_aCo_bFe_cBi_dP_eMo_fO_x$, in which a represents a number from 2 to 20, b represents a number from 0 to 15, a and b represent a number from 2 to 20, c represents a number from 0.1 to 7, d represents a number from 0.1 to 4, e represents a number from 0.1 to 4, f represents approximately 12 and x represents a number from 35 to 85, and also consisting of from 0.2 to 5 % by weight of tantalum or samarium, expressed as $Ta_2O_5$ or $Sm_2O_3$, and optionally also from 0.05 to 3.0 % by weight of alkali- or alkaline-earth metal, expressed as oxide, on a carrier material consisting of a laminar silicate and/or highly dispersed silicon dioxide — in the first case in a weight ratio of from 10 : 1 to 1 : 1 — the shell being obtained by spraying a suspension of the shell starting material onto moving loose carrier material while some of the suspending agent is drawn off by a flow of gas at from 20 to 250 °C, while maintaining a substantially constant residual moisture of the shell, and by drying and annealing operations, characterised in that shell catalysts are used, for the preparation of which the loose carrier material is set in a mixing motion by mechanical means and is simultaneously loosened by the injection of a fluidising flow of gas from below which intensifies the mixing action, the suspension, containing a binder and optionally a pore-former, of a precursor of the catalytically active material is supplied to this loose material in a counter flow to the gas, in a quantity which increases as the thickness of the shell increases, and the quantities of the suspending agent which are moved and sprayed are maintained in a substantially constant ratio, which is determined by the combinations of carrier and precursor used in each case, and the thermal expansion coefficients of carrier and dried precursor in powder from are coordinated such that they differ by 15 % at the most, and that after spraying, the shell solidifies by the continuation of the intensified mixing motion, the mechanical mixing motion is then stopped, the material is dried in the continually flowing gas and is finally annealed preferably over a period of from 0.05 to 5 hours at from 520 to 650 °C, optionally after the decomposition of an added pore former.

2. A process according to claim 1, characterised in that the shell of the shell catalyst which is used is applied and dried in an apparatus in accordance with DE-OS 2 805 801.

3. A process according to claim 1 or 2, characterised in that the shell catalyst is obtained by adjusting the fluidising flow of gas to a specific throughput of from 15 to 50 $Nm^3$ per hour and per litre of carrier, and by using water as the suspending agent.

4. A process according to claims 1 to 3, characterised in that the shell catalyst is obtained using a suspension which consists of from 20 to 80, preferably from 40 to 70, in particular 55 % by weight of the precursor in powder form.

5. A process according to claims 1 to 4, characterised in that the shell catalyst is obtained using a suspension which contains from 2 to 5, in particular about 4 % by weight of glucose or urea as a binder.

6. A process according to claims 1 to 5, characterised in that the shell catalyst is obtained using a suspension of the starting material in powder form for the shell which contains from 1 to 10 % by weight, based on the weight of this material, of a finely-divided pore former which is difficultly-soluble in the suspending agent, and which was removed by thermolysis or oxidation at a temperature below the annealing temperature.

7. A process according to claims 1 to 6, characterised in that the shell catalyst is obtained using a precursor consisting of a dried co-precipitate or a co-precipitate which is calcined at a temperature below the annealing temperature, consisting of combined salt solutions of the catalytically active elements.

8. A process according to claims 1 to 7, characterised in that the precursor of the shell catalyst which is used is a powder having a grain size distribution of from 1 to 150 $\mu$m with a preferred maximum in the range of from 1.5 to 30 $\mu$m.

9. A process according to claims 1 to 8, characterised in that the quantity of the precursor in powder form of the shell catalyst which is used is from 0.1 to 2 times the weight of the carrier.

10. A process according to claims 1 to 9, characterised in that the carrier of the shell catalyst which is used is $\alpha$-aluminium oxide, aluminium silicate, magnesium silicate or silicon carbide.

11. A process according to claim 10, characterised in that the roughness of the surface of the carrier has an average roughness value of from to 50 $\mu$m, according to DIN 4768/1, measured using Hommels instrument for measuring roughness-depth.

12. A process according to claims 1 to 11, characterised in that, for the preparation of the shell catalyst, the thermal expansion coefficient of the precursor powder is coordinated with that of the carrier by a preliminary thermal treatment at from 250 to 600 °C.

13. A process according to claims 1 to 12, characterised in that the oxygen-containing gas mixture which is supplied to the oxidation reaction on the shell catalyst has a molar ratio of olefin or alcohol to oxygen and diluting, substantially inert, components of from 1 : 1.5-2.5 : 7-20.

14. A process according to claim 13, characterised in that the oxygen-containing gas mixture, contains nitrogen, water vapour, oxides of carbon and saturated aliphatic hydrocarbons having from 1 to 5 carbon atoms or mixtures thereof as diluting, substantially inert, components.

15. A process according to claims 1 to 14, characterised in that the oxidation of propylene to produce acrolein is carried out at a molar ratio of propylene : oxygen nitrogen : water vapour of from 1 : 1.5-1.8 : 5.5-7 : 2-4 and at a specific load of from 2 to 6 mols of propylene per kg of the shell catalyst · h.

16. A process according to claims 1 to 14, characterised in that the oxidation of propylene to produce acrolein is carried out with the partial to complete substitution of a charging of water vapour by an inert gas mixture in a molar ratio of propylene : air : inert gas : water of from 1 : 7-9 : 3-8 : 0-2 and at a specific load of from 2 to 6 mols of propylene per kg of the shell catalyst · h.

17. A process according to claim 16, characterised in that the waste gas from the reaction freed from acrolein, acrylic acid and other components which may be condensed in a temperature range of from 0 to 40 °C, is used as inert gas.

18. Process according to claim 16 or 17, characterised in that the inert gas contains from 0.5 to 7 % by weight volume of water vapour.

19. A process according to claims 1 to 14, characterised in that the oxidation of isobutene or tertiary butanol to produce methacrolein is carried out in molar ratio of isobutene or tertiary butanol : oxygen : inert gas : water vapour of from 1 : 1.5-2.5 : 5.5-10 : 2-10 and at a specific load of from 1 to 6 mols of the starting compound per kg of shell catalyst · h.

**Revendications**

1. Procédé pour la préparation de l'acroléine ou de la méthacroléine par oxydation du propylène, de l'isobutylène ou du butanol tertiaire, dans des mélanges gazeux contenant de l'oxygène, sur un catalyseur en coquille résistant à l'abrasion, formé d'un support inerte à surface rugueuse, avec une grosseur de grains de 0,5 à 6 mm et d'une coquille en catalyseur actif qui enveloppe ce support et qui y est fixée, la composition du catalyseur étant $Ni_aCo_bFe_cBi_dP_eMo_f$-$O_x$, où a signifie un nombre de 2-20, b un nombre de 0-15, a et b un nombre de 2-20, b un nombre de 0-15, a et b un nombre de 2-20, c un nombre de 0,1-7, d un nombre de 0,1-4, e un nombre de 0,1-4, f un nombre à peu près égal à 12, et x un nombre de 35-85, ainsi qu'en plus, 0,2 à 5 % en poids de tantale ou de samarium, calculé en $Ta_2O_5$ ou $Sm_2O_3$, ainsi qu'éventuellement encore 0,05-3 % en poids de métal alcalin ou alcalino-terreux, calculé en oxyde, sur une substance support en silicate stratifié et/ou en dioxyde de silicium hautement dispersé — dans le premier cas, dans un rapport en poids de 10 : 1 à 1 : 1 — la coquille étant obtenue par pulvérisation d'une suspension du matériau de départ de la coquille sur un support en vrac en mouvement, avec enlèvement partiel de l'agent de mise en suspension au moyen d'un courant gazeux à 20-250 °C, avec maintien d'une humidité résiduelle essentiellement constante de la coquille, ainsi que par séchage et traitement thermique, procédé caractérisé en ce que l'on met en œuvre des catalyseurs en coquilles pour la préparation desquels le support en vrac est soumis à un mouvement de mélange par action mécanique et est simultanément ameubli de bas en haut par le passage d'un courant gazeux fluidisant, renforçant l'action de mélange, à cette matière en vrac est amenée, à contre-courant du gaz, une suspension, contenant un agent liant et éventuellement une substance formatrice de pores, d'une étape préliminaire ou ébauche de la matière catalytiquement active, dans une proportion augmentant avec l'accroissement de l'épaisseur de la coquille, les proportions d'agent de suspension enlevé et pulvérisé étant maintenues dans un rapport constant qui est déterminé par les proportions de la combinaison de support et d'ébauche de catalyseur utilisée chaque fois, le coefficient de dilatation thermique du support et celui de l'ébauche pulvérulente séchée étant ajustés de façon à ne différer que de 15 % au plus, qu'après la fin de la pulvérisation, la coquille est rendue plus compacte par la poursuite du mouvement de mélange renforcé, puis on arrête le mouvement de mélange mécanique, sèche la matière dans le courant gazeux continuant à affluer et enfin, la soumet à un traitement thermique, éventuellement après décomposition d'un formateur de pores que l'on aura ajouté, de préférence pendant 0,05-5 heures à 520-650 °C.

2. Procédé selon la revendication 1, caractérisé en ce que la coquille de catalyseur mise en œuvre est déposée dans un dispositif selon DE-OS 2 805 801, puis séchée.

3. Procédé selon l'une des revendications 1 ou 2, caractérisé en ce que le catalyseur-coquille est obtenu en ajustant le courant gazeux fluidisant à un débit spécifique de 15-50 $m^2$ par heure et par litre de support, et en utilisant de l'eau comme agent de mise en suspension.

4. Procédé selon l'une quelconque des revendications 1 à 3, caractérisé en ce que le catalyseur-coquille est obtenu par mise en œuvre d'une suspension, qui est constituée par 20 à 80, de préférence 40 à 70, en particulier 55 % en poids de l'ébauche pulvérulente.

5. Procédé selon l'une quelconque des revendications 1 à 4, caractérisé en ce que le catalyseur-coquille est obtenu par mise en œuvre d'une suspension contenant comme agent liant 2 à 5, en particulier environ 4 % en poids de glucose ou d'urée.

6. Procédé selon l'une quelconque des revendications 1 à 5, caractérisé en ce que le catalyseur-coquille est obtenu par mise en œuvre d'une suspension de la matière de départ pulvérulente de la coquille, qui contient 1-10 % en poids, calculé sur le poids de cette matière, d'un formateur de pores finement divisé difficilement soluble dans l'agent de suspension, qui est éliminé ensuite par thermolyse ou oxydation à une température inférieure à celle du traitement thermique.

7. Procédé selon l'une quelconque des revendications 1 à 6, caractérisé en ce que le catalyseur-coquille est obtenu par mise en œuvre d'une ébauche constitué par un co-précipité, séché et calciné à une température inférieure à celle du traitement thermique, des solutions salines unifiées des éléments catalytiquement actifs.

8. Procédé selon l'une quelconque des revendications 1 à 7, caractérisé en ce que l'ébauche du catalyseur-coquille mise en œuvre est une poudre avec une répartition granulométrique de 1-150 μ, avec un maximum situé de préférence dans la zone 1,5 à 30 μ.

9. Procédé selon l'une quelconque des revendications 1 à 8, caractérisé en ce que la proportion de l'ébauche pulvérulente du catalyseur-coquille mis en œuvre se monte à 0,1 à 2 fois le poids du support.

10. Procédé selon l'une quelconque des revendications 1 à 9, caractérisé en ce que le support du catalyseur-coquille est constitué par de l'oxyde d'aluminium α, du silicate d'aluminium, du silicate de magnésium ou du carbure de silicium.

11. Procédé selon la revendication 10, caractérisé en ce que la rugosité de la surface du support présente une valeur moyenne de 5 à 50 μ selon DIN 4768/1, mesuré avec l'appareil à mesurer la profondeur de rugosité selon Hommel.

12. Procédé selon l'une quelconque des revendications 1 à 11, caractérisé en ce que pour préparer le catalyseur-coquille, le coefficient de dilatation thermique de la poudre d'ébauche est accordé avec celui du support par un prétraitement thermique à 250-600 °C.

13. Procédé selon l'une quelconque des revendications 1 à 12, caractérisé en ce que le mélange gazeux contenant de l'oxygène amené à la réaction d'oxydation sur le catalyseur-coquille présente un rapport molaire entre l'oléfine ou l'alcool, l'oxygène et les composants, dans une large mesure inertes servant à diluer, de 1 : 1,5 à 2,5 : 7 à 20.

14. Procédé selon la revendication 13, caractérisé en ce que le mélange gazeux contenant de l'oxygène contient, comme composants servant à diluer, dans une grande mesure inertes, de l'azote, de la vapeur d'eau, de l'oxyde de carbone et des hydrocarbures aliphatiques saturés avec 1 à 5 atomes de C, ou un mélange de ces produits.

15. Procédé selon l'une quelconque des revendications 1 à 14, caractérisé en ce que l'oxydation du propylène en acroléine s'effectue avec un rapport molaire propylène : oxygène : azote : vapeur d'eau de 1 : 1,5 à 1,8 : 5,5 à 7 : 2 à 4, et sous une charge spécifique de 2 à 6 moles de propylène/kg de catalyseur-coquille/heure.

16. Procédé selon l'une quelconque des revendications 1 à 14, caractérisé en ce que l'oxydation du propylène en acroléine est effectuée avec remplacement partiel ou total de l'introduction de vapeur d'eau par l'introduction d'un mélange gazeux inerte avec un rapport molaire propylène : air : gaz inerte : eau de 1 : 7 à 9 : 3 à 8 : 0 à 2 et sous une charge spécifique de 2 à 6 moles de propylène/kg de catalyseur-coquille/h.

17. Procédé selon la revendication 16, caractérisé en ce que comme gaz inerte on utilise, le gaz de rejet de la réaction, libéré de l'acroléine, de l'acide acrylique et des autres composants condensables dans une zone de température de 0 à 40 °C.

18. Procédé selon l'une des revendications 16 ou 17, caractérisé en ce que le gaz inerte contient 0,5-7 % en volume de vapeur d'eau.

19. Procédé selon l'une quelconque des revendications 1 à 14, caractérisé en ce que l'oxydation de l'isobutène ou du butanol tertiaire en méthacroléine s'effectue avec un rapport molaire isobu-tène ou butanol tertiaire : oxygène : gaz inerte : vapeur d'eau de 1 : 1,5 à 2,5 : 5,5 à 10 : 2 à 10, et sous une charge spécifique de 1-6 moles de composé de départ/kg de catalyseur-coquille × h.